# EUROPEAN PATENT APPLICATION

(11) **EP 3 073 399 A1**
(43) Date of publication of application: **28.09.2016**
(21) Application number: 16159286.0
(22) Date of filing: 08.03.2016
(51) Int. Cl.: G06F 19/00

(54) **SYSTEM AND METHOD FOR PROVIDING INDIVIDUALIZED HEALTH AND WELLNESS COACHING**

(30) Priority: 25.03.2015 US 201514668682
(71) Applicant: Palo Alto Research Center, Incorporated, Palo Alto, CA 94304 (US)
(72) Inventor: RAM, Ashwin, Palo Alto, CA 94306 (US); NELSON, Lester D., Santa Clara, CA 95050 (US); YOUNGBLOOD, Gregory Michael, San Jose, CA 94116 (US); PIROLLI, Peter L., San Francisco, CA 94116 (US)
(74) Representative: Gill Jennings & Every LLP

(57) **Abstract**

One embodiment of the present invention provides a system for generating healthcare suggestions. During operation, the system extracts data based on a user's communication, which can be between the user and other users or presented online by the user. The system identifies a health-related issue from the extracted data. The system then generates, based on the extracted data, content that indicates a first suggestion corresponding to the health-related issue. The system subsequently monitors the user's communication to generate an additional suggestion that is an improvement over the first suggestion.

## Description

### BACKGROUND

### Field

The present disclosure relates to individualized health and wellness coaching. More specifically, this disclosure relates to a method and system for generating customized healthcare suggestions and programs by crowdsourcing user-generated goals, activities, and conversations.

### Related Art

There are a number of programs that help users to improve health and wellness, including programs that assist with diets, exercise programs, and fitness regimen. However, "one size fits all" programs are not practical or effective for a broad range of people. Users vary in their dietary needs, physical fitness levels, availability to exercise, and in other ways. It is difficult, expensive, and impractical to manually list or author all possible variations, substitutions, and customizations for each aspect of a program. Furthermore, although a skilled personal coach can tailor goals, activities, and coaching interventions to each individual, many people do not have access to human coaches for reasons of time, location, or cost.

### SUMMARY

One embodiment of the present invention provides a system for generating healthcare suggestions. During operation, the system extracts data based on a user's communication, which can be between the user and other users or presented online by the user. The system identifies a health-related issue from the extracted data. The system then generates, based on the extracted data, content that indicates a first suggestion corresponding to the health-related issue. The system subsequently monitors the user's communication to generate an additional suggestion that is an improvement over the first suggestion.

In a variation on this embodiment, the system determines qualifications for a plurality of users that can be accessed through a crowdsourcing platform. Next, the system solicits feedback from the plurality of users regarding the health-related issue. The system receives feedback from the plurality of users. The system then generates, based on the feedback, suggested content for addressing the health-related issue. The system receives input from one or more users for evaluating the suggested content to validate the suggested content, and stores validated suggestions in a validated suggestions storage.

In a variation on this embodiment, the system presents a candidate substitution to a user as conversational content, and receives the user's vote on whether the user likes the candidate substitution.

In a variation on this embodiment, the system generates validated suggestions by identifying health problems and extracting solutions and health advice with ongoing monitoring of one of sensor data, website forums, instant messages, websites, and social media communication.

In a variation on this embodiment, the system generates a healthcare question and posts the healthcare question on a website forum to solicit responses from forum users. The system monitors responses to the healthcare question on the website forum, and generates a suggested solution addressing the healthcare question based on the responses to the posted healthcare question.

In a variation on this embodiment, the system receives a query from a user. The system executes the query to determine similarity matches for one or more pairs of webpages. Next, the system displays pairs of webpages with similarity matches that exceed a predetermined threshold. The system receives user input to examine a pair of webpages and to edit recommended suggestions, and generates a validated suggestion based on the user input.

In a variation on this embodiment, the system generates a program that provides one or more suggestions to perform actions affecting a health-related issue.

### BRIEF DESCRIPTION OF THE FIGURES

FIG. 1 presents a block diagram illustrating an exemplary architecture of an individualized health and wellness coaching system, according to an embodiment.
FIG. 2 presents a flowchart illustrating an exemplary process for generating healthcare suggestions, according to an embodiment.
FIG. 3 presents a flowchart illustrating an exemplary process for accessing web content to generate validated suggestions, according to an embodiment.
FIG. 4 presents a flowchart illustrating an exemplary process of a workflow for authoring content by content authors, according to an embodiment.
FIG. 5 presents an illustration of a content-authoring website displaying analysis of text chunks across webpage pairs that each match a search query, according to an embodiment.
FIG. 6 presents an illustration of results of similarity analysis for a pair of webpages to provide initial content recommendation, according to an embodiment.
FIG. 7 presents an illustration of displaying a recommended content selection in the context of other contiguous text to receive user selection for content generation, according to an embodiment.
FIG. 8 presents a flowchart illustrating an exemplary process for using crowdsourcing and collaborative filtering to provide validated suggestions, according to an embodiment.
FIG. 9 presents a block diagram illustrating an exemplary apparatus for personalizing healthcare suggestions, in accordance with an embodiment.
FIG. 10 illustrates an exemplary computer system that may personalize healthcare suggestions, in accordance with an embodiment.

In the figures, like reference numerals refer to the same figure elements.

### DETAILED DESCRIPTION

The following description is presented to enable any person skilled in the art to make and use the embodiments, and is provided in the context of a particular application and its requirements. Various modifications to the disclosed embodiments will be readily apparent to those skilled in the art, and the general principles defined herein may be applied to other embodiments and applications without departing from the spirit and scope of the present disclosure. Thus, the present invention is not limited to the embodiments shown, but is to be accorded the widest scope consistent with the principles and features disclosed herein.

### Overview

Embodiments of the present invention solve the problem of providing personalized healthcare advice and coaching to users by mining web data and/or using crowdsourcing and collaborative filtering to generate personalized healthcare-related suggested content. These suggestions are typically actionable advice that a user can follow to improve their health or maintain a healthy and active lifestyle. The suggestions may include, for example, substitutions, variations, or customizations of goals, activities, and/or coaching tips.

This disclosure describes a system and method for creating a library of program customizations through automated techniques that monitor, analyze, model and summarize user-generated goals, activities, and conversations. An individualized health and wellness coaching system as disclosed herein can automatically create libraries of health and wellness goals, activities, and coaching tips, using the input of users while users are engaged with a health and wellness program.

The system may personalize health and wellness goals, activities, and coaching tips. For example, vegetarian users may substitute a recommended goal to consume fish oil with a goal to consume flaxseed oil. The system can learn this substitution, analyze the group of users who have been making this substitution, and recommend it to other users with similar constraints. The customizations may also involve health and wellness activities. For example, users may substitute a jogging activity with a bike ride. The system can model this substitution and recommend it as part of other programs involving jogging or running. Customizations can include coaching tips. For example, users may suggest adding spinach to a morning omelette as a way to increase vegetable servings. The system can generalize this tip and include a suggestion to add vegetables to breakfast.

The system may use crowdsourcing and collaborative filtering, as well as intelligent data mining of websites and other data sources, to identify health-related problems and generate suggested solutions. Crowdsourcing is a process for obtaining content or ideas by soliciting input from a group of people. Usually the group of people is comprised of users of an online community. Collaborative filtering is a technique for automatically predicting the interests of a user by collecting preference data from users with similar preferences. Some examples of the other data sources that the system can data mine include communications among users such as discussion forums and social media postings, activity data, and other data sources on the web. The system may also continuously monitor the sources of data to improve the suggested content and identify new problems.

The system can proactively interact with the data sources or passively monitor the data sources. For example, the system can proactively post questions to a website forum or to a crowdsourcing platform such as Amazon Mechanical Turk to elicit responses that the system can extract data from. Amazon Mechanical Turk is a marketplace where one can crowdsource tasks by posting the tasks for people to work on, and human workers can collect a reward for completing each task. Similarly, the system can passively monitor communications among users and extract problems or solutions including suggested content from postings or conversations.

By automatically learning and generating suggestions, and improving the suggestions, the system frees human administrators and developers from the need to manually generate suggestions or programs. The system can automatically run by itself and improve itself over time.

In one implementation, a group of users seeking a team-based health challenge may each install a mobile application on their respective devices. The mobile application is a mobile behavior change platform and health challenge exchange. The users choose from a variety of health challenges to accomplish their respective goals. The users engage in personalized user experiences and receive healthcare suggestions from an intelligent coaching agent. Users select a health challenge from the exchange and join a team. The coaching agent learns about the team and its members as the team embarks on the chosen challenge. The agent provides personalized timely advice, encouragement, and feedback. Users may also communicate with each other within the mobile application. The user experience continues to improve and become more personalized as the mobile application learns more about each user. This disclosure describes the aspects of the system that involve generating, evaluating, and storing suggestions that the coaching agent can provide to the users.

### System Architecture

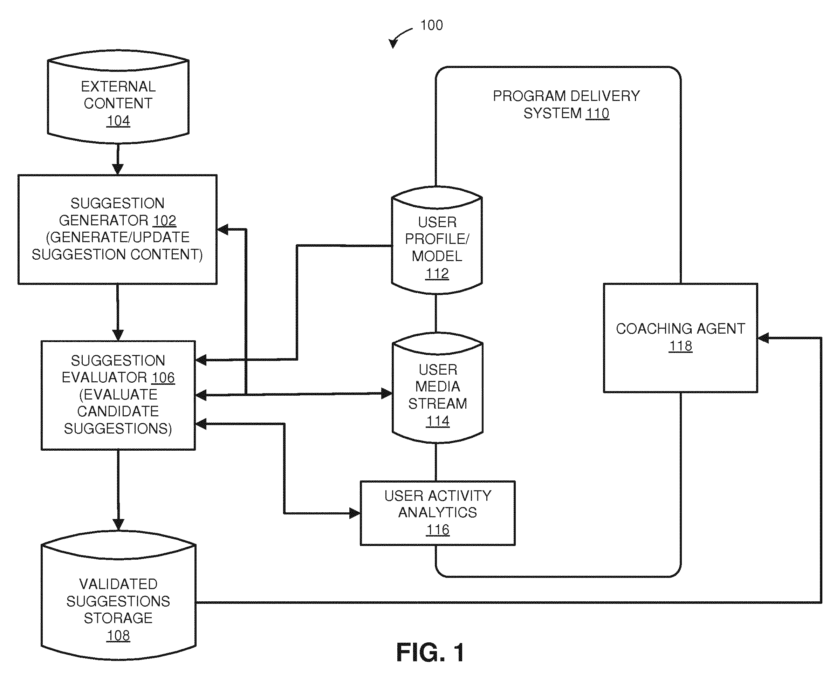

FIG. 1 presents a block diagram illustrating an exemplary architecture of an individualized health and wellness coaching system 100, according to an embodiment. As illustrated in FIG. 1, system 100 includes suggestion generator 102 (which accesses external content 104), suggestion evaluator 106, and validated suggestions storage 108 for generating and evaluating candidate suggestions, and storing validated suggestions. System 100 also includes program delivery system 110 with user profile/model 112, user media stream 114, user activity analytics 116, and coaching agent 118. These components perform functions that include analyzing and storing user activity, user profile and user communications (e.g., social media postings and/or activity streams), and delivering validated suggestions. The details of the components are provided below.

System 100 includes suggestion generator 102 for generating and/or updating candidate suggestions. The suggestion content is typically actionable guidance, instruction, or advice. Suggestion generator 102 may access external content 104 and/or user media stream 114 and/or user profile/model 112 to generate health-related suggestions. External content 104 may include web content, such as webpages, social bookmarking services, crowdsourcing services, and other sources of information. System 100 also includes suggestion evaluator 106 to facilitate evaluating candidate suggestions. In some implementations, suggestion evaluator 106 may allow users to select, arrange, edit, and share content with others to validate suggestions. Suggestion evaluator 106 stores validated candidate suggestions in validated suggestions storage 108.

Program delivery system 110 includes components that store user profiles and user media streams, analyze user activity, and deliver personalized suggestions to a respective user. Program delivery system 110 includes storage for user profile/model 112 and storage for user media stream 114. In some implementations, user media stream 114 includes historical and current social media postings, and system 100 may generate and/or update suggested content based on data from user media stream 114 and/or user profile/model 112. Suggestion generator 102 and/or suggestion evaluator 106 may read from and/or write to user profile/model 112 and user media stream 114 to generate and evaluate suggested content.

Program delivery system 110 also includes user activity analytics 116 and coaching agent 118. User activity analytics 116 analyzes the user activity and may also access data from suggestion evaluator 106 as part of analyzing user activity. Suggestion evaluator 106 may also interact with user activity analytics 116 to evaluate and validate suggestions. Coaching agent 118 learns about team members and provides encouragement and feedback. Coaching agent 118 may retrieve validated suggestions from validated suggestions storage 108 and communicate the suggestions to the user.

System 100 is typically implemented on a mobile device, although in some implementations, some components such as suggestion generator 102, suggestion evaluator 106 and/or validated suggestions storage 108 may be implemented on a server communicating with a user's mobile device. Note that most or all components of program delivery system 110 are installed on the user's mobile device.

### Generating Suggestions and Other System Capabilities

In order to provide personalized healthcare suggestions, a content-generation system such as system 100 applies a variety of techniques to generate, validate and store suggestions. System 100 can apply any combination of a selection of methods to generate a set of suggestions relating to healthcare. The system may passively monitor and perform data mining on live user communications and interactions with others to extract and store healthcare suggestions. The system may also data mine websites with online forums to extract solutions or other content from online discussions. The system may automatically crawl webpages, such as static webpages providing healthcare information, to extract healthcare suggestions. For example, the system may extract suggestions for back injury exercises from a webpage that provides exercises for back injuries. The system may also search websites for suggestions or solutions to problems, in response to determining that a user has a specific problem.

System 100 may monitor social media communications to extract and generate suggestions. System 100 can monitor a group of users that are using social media to communicate with each other to extract and encapsulate suggestions. For example, a group of friends may walk together to motivate each other to exercise. A user may be feeling pain in his legs from the exercise and communicate with others to seek advice on how to exercise without pain. The user may be operating a mobile application with system 100 executing on his mobile device. The user may use social media to communicate with other users and receive advice. For example, other users may suggest variations in the exercise, such as that the user may walk on flat land, get specialized walking shoes, or stretch and take breaks every 30 minutes. System 100 may data mine the suggestions of the other users to generate candidate suggestions, evaluate candidate suggestions, and store validated suggestions in validated suggestions storage 108. The next time another person has a similar problem, system 100 can provide the suggestion to the other person. System 100 may provide the other person with a substitute exercise technique extracted from monitoring previous communications among different users.

Note that besides monitoring social media communications for feedback from the other users, system 100 may also monitor SMS communications or other social networking-type services. In some implementations, system 100 can monitor live voice communications using microphones and generate candidate suggestions.

System 100 may also use a hybrid method of text mining the web (or other document sources) for basic propositions that the system then presents to users (or other crowd sources) for evaluating to determine suggestions. For example, a user may be interested in searching for tips on how to cook with quinoa as a substitute for other ingredients. The user may perform a Google search for "what should I substitute for quinoa," which produces a first search result of http://www.3fatchicks.com/how-to-easily-substitute-dishes-with-quinoa/. Parsing this text yields foods related to quinoa, and the system may determine that one can directly substitute rice or couscous with quinoa. The system may also determine that quinoa flour is gluten-free, which means that quinoa flour can be excellent for gluten-free diets, but it cannot always be used as a direct substitute for wheat flour in baking.

The system may present these candidate substitutions to users of program delivery system 110 as conversational content and the users may use the annotation features of that system for evaluations. For example, users may vote for a substitution, indicate whether they like a substitution or not, and/or comment on a substitution. Note that conversational content may include chat or other group communication media streams, individual communication streams that may be addressed to recipients by a user model (e.g., interest tags in a profile, topic models based on prior conversations, etc.), and online game delivery such as quiz games. Furthermore, system 100 may also present suggested content other than substitutions to the user, and may also obtain suggestions from users by providing choices or other customization opportunities within the program delivery system. Also, some implementations may incorporate similar hybrid methods using the techniques described herein in the program delivery system.

The system may also actively pose a query to a crowd, using a crowdsourcing Internet marketplace such as Amazon Mechanical Turk or an online forum, such as WebMD discussion forums. The system may post problems and receive solutions from the crowd, and generate suggestion content from the answers or discussions received from the crowd. System 100 can post questions on social forums or monitor interactions among forum users in order to extract, evaluate, and store suggestions.

Some implementations may include a method for users to record substitutions while selecting or engaging in a health and wellness program. For example, the system and/or the users may record substitutions when users select from a list of choices or write in a new choice when available choices are not appropriate. The system may also record substitutions when users engage in text conversations with other users as part of a forum or social network.

System 100 may also query a fitness-tracking application installed on a mobile device for behavior traces, including a user's running or walking activities. These fitness-tracking applications may use the Global Positioning System (GPS) to track the user's physical activities. System 100 may data mine the activity data from the fitness-tracking application to generate candidate suggestions. For example, if system 100 extracts data indicating that an average person usually follows a long run of x miles with a walk the next day, system 100 may generate a suggestion with this information for other runners.

System 100 may also collect execution metrics for a user to determine the extent to which the user successfully follows through with a suggestion. The system may evaluate the extent to which the suggestion is working to solve a problem, and system 100 may calibrate one or more suggestions based on the evaluation. For example, the system may evaluate the extent to which a suggestion addresses a user's health concerns and change the suggestion or leave the suggestion unchanged, and may also modify other suggestions.

In some implementations, the system may perform any combination of crawling static webpages, scanning online forums, monitoring live discussions, posing queries to a crowd (e.g., text messages, microblogging, or other types of instant communication), and/or any other techniques disclosed herein to extract suggestions that the system validates and stores in a validated suggestions storage.

In some implementations, the system may also generate a program. This program may be an application or some other executable file. For example, the system may determine that many users have sleeping problems, and the system lacks a sleep coaching program. The system may automatically crawl the web, discussion forums, and/or other information sources to extract data and generate a sleep coaching program based on the extracted data. Since users may have different medical problems and lifestyles, the system may generate different coaching programs for different users. The system may also improve the sleep coaching program through usage over time.

Note that the system may independently identify challenges or problems and provide a suggested solution or program. The system can independently (e.g., without explicit prompting by a user) determine the problem or suggested solution. The system can, without explicit prompting by the user, suggest actionable solutions to a user. For example, the system can suggest to the user actions that the user can take to improve the user's lifestyle in healthy ways, or the system can suggest to the user actions for improving the user's active lifestyle or quality of life.

In some implementations, system 100 may apply techniques to analyze and summarize user-generated inputs. This may involve statistical analysis (e.g., frequency of suggestions), text mining (e.g., analysis of write-in choices), or natural language processing (e.g., analysis of short text conversations).

In some implementations, system 100 may apply techniques to model groups of users for whom particular suggestions are applicable. This may include statistical modeling or machine learning techniques, such as clustering, applied to groups of users who have provided or selected similar substitutions.

### Generating Healthcare Suggestions

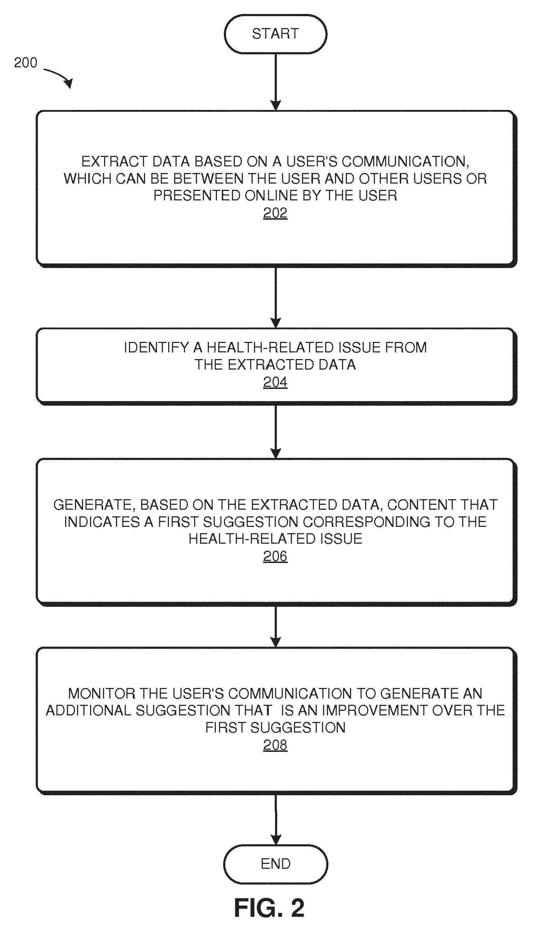

FIG. 2 presents a flowchart 200 illustrating an exemplary process for generating healthcare suggestions, according to an embodiment. During operation, system 100 extracts data based on a user's communication, which can be between the user and other users or presented online by the user (operation 202). Next, system 100 identifies a health-related issue from the extracted data (operation 204). System 100 then generates, based on the extracted data, content that indicates a first suggestion corresponding to the health-related issue (operation 206). Subsequently, system 100 monitors the user's communication to generate an additional suggestion that is an improvement over the first suggestion (operation 208).

### Accessing Web Content to Generate Validated Suggestions

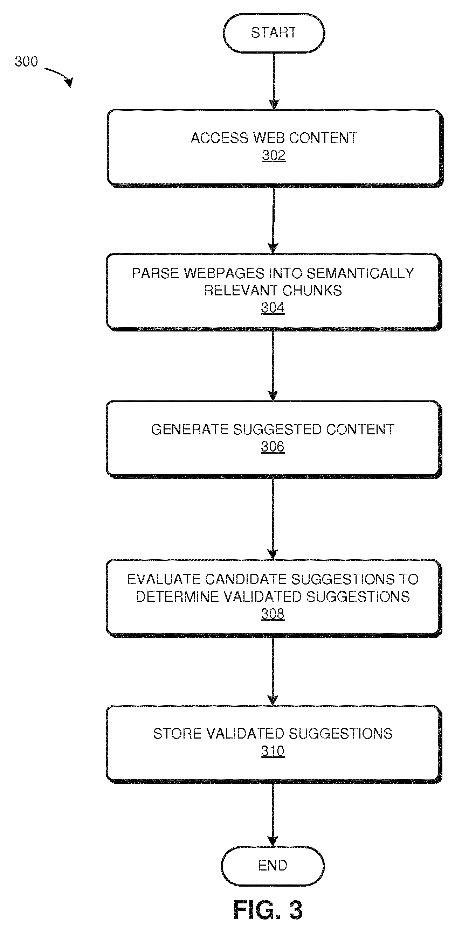

FIG. 3 presents a flowchart 300 illustrating an exemplary process for accessing web content to generate validated suggestions, according to an embodiment. FIG. 3 illustrates one possible implementation, and specific details may vary according to implementation. System 100 may apply the process of FIG. 3 to generate or update validated suggestions. During operation, system 100 may initially access web content as external content pages through a web search or a social bookmarking service (operation 302). For example, system 100 may perform a web search through google.com or bing.com, or system 100 may access webpages through a social bookmarking service (e.g., http://en.wikipedia.org/wiki/Delicious_%28website%29).

System 100 may parse the webpages into semantically relevant chunks (operation 304). System 100 may put natural language sentences into canonical forms, including applying one or more of consistent case, stop word removal, bag of word representation (e.g., unique words, word frequency, original word ordering), and substitution to a root word. Substitution to a root word may include use of common abbreviations, word stemming, and use of a word similarity function, such as synonyms (e.g., thesaurus or WordNet® synsets).

System 100 may also parse the webpages by generating *n*-grams of extracted words or words in a canonical form, or generate semantically relevant chunks that include text elements accessed by web document structure (e.g., by HTML tag H1, H2, LI, TD, etc.).

System 100 may then generate suggested content using text analytical approaches (operation 306). For example, system 100 may determine the cosine similarity of content chunks used across webpages (e.g., pairwise comparison of chunks relating to top-ranked search results in a web search). System 100 may also analyze *n*-gram distributions, including counts across webpages and order sequences such as near neighbors, common orderings across multiple webpages, or following or preceding specific HTML tags (e.g., first H1 occurrence). System 100 may also use combined chunking approaches such as *n-*gram analysis within canonical sentences.

In some implementations, the content recommendation process may include other editing capabilities, such as aggregating selections across multiple types of analyses of webpages (e.g., pair-wise similarity, *n*-gram selection) and using patterns in a history of selections across queries to augment future recommendations. One example of this use is that the system can suppress selections that are routinely deleted across queries in future presentations (e.g., the system recognizes that a phrase like "Website Created & Managed By" is never included by anyone, and ignores the phrase thereafter).

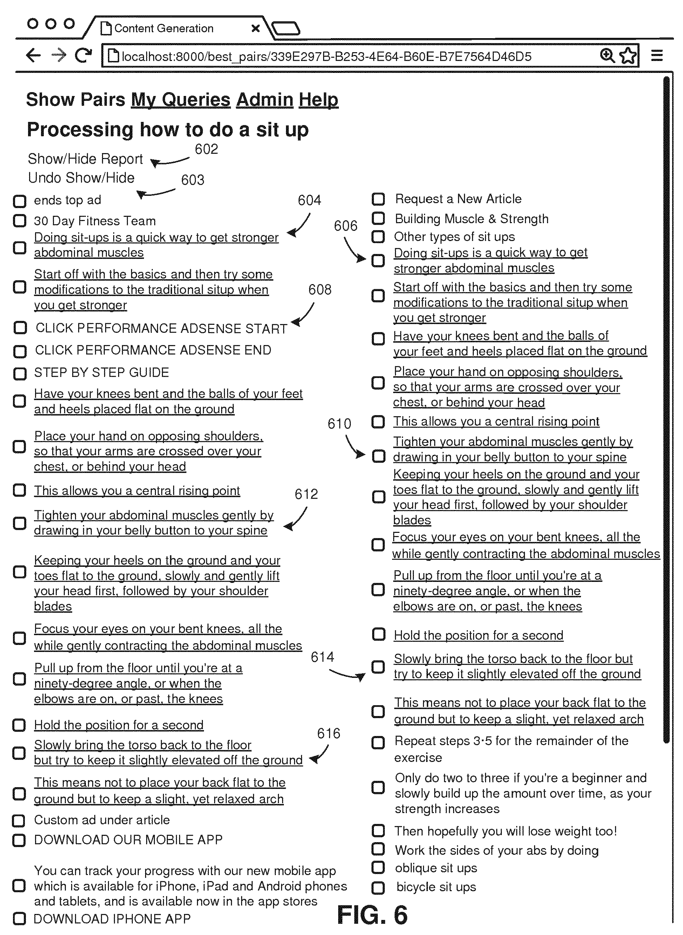

System 100 may facilitate evaluation of the candidate suggestions to determine validated suggestions (operation 308). System 100 may assemble content chunks into a shared environment, which allows interested parties (e.g., subject matter experts, health and wellness program participants) to select, arrange, and edit content. For example, FIGs. 6-8 depict an implementation of a content-authoring website that presents pairwise comparison of chunks relating to top-ranked search results in a web search, and allows users of the content-authoring website to select, arrange, and share those selections with others. The outcome of this interaction is one or more validated suggestions.

System 100 may store validated suggestions in validated suggestions storage 108 (operation 310). Program delivery system 110 may access the validated suggestions from validated suggestions storage 108 and present the validated suggestions to the mobile device user.

### Authoring Content

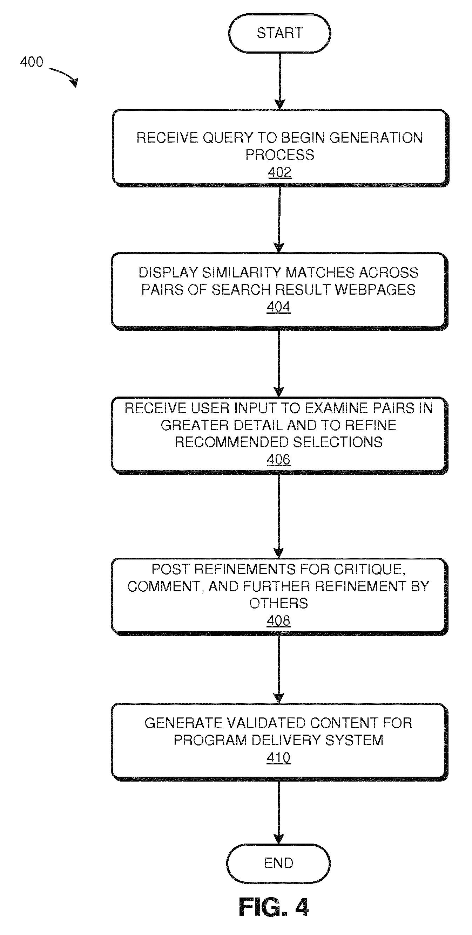

FIG. 4 presents a flowchart 400 illustrating an exemplary process of a workflow for authoring content by content authors, according to an embodiment. FIG. 4 illustrates one possible implementation, and specific details may vary according to implementation. Some implementations may allow a content author, such as a subject matter expert, or health and wellness program participants, to use a content-generation system such as system 100 to generate content.

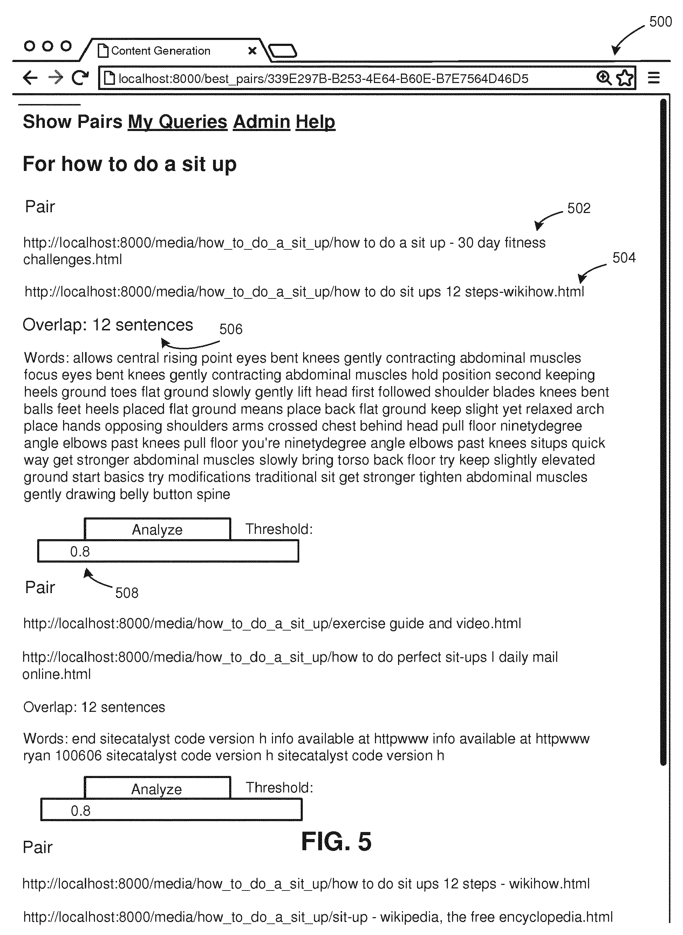

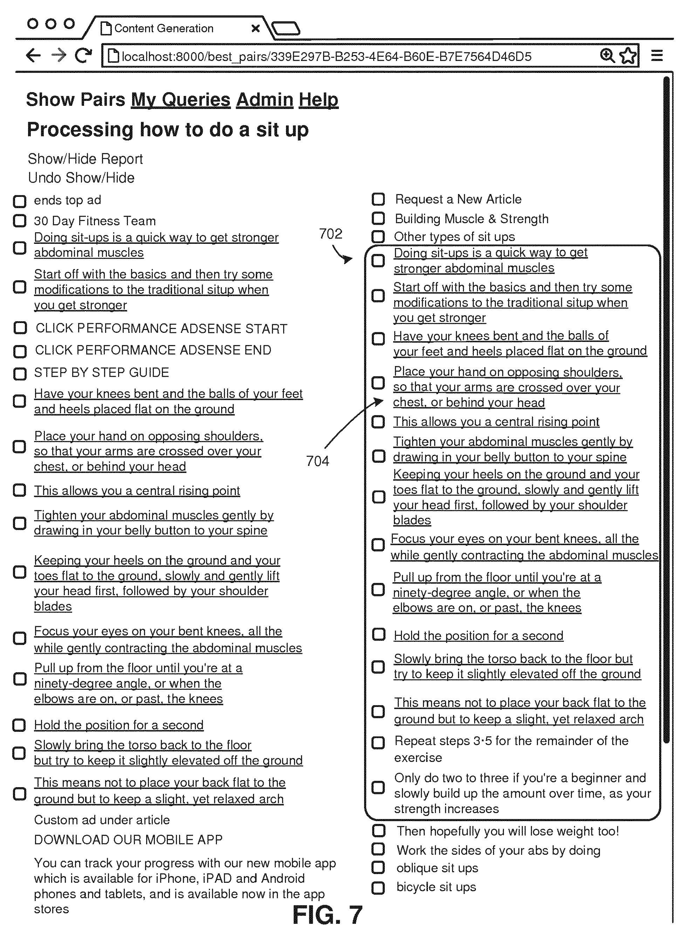

During operation, system 100 may initially receive a query to begin a generation process (operation 402). System 100 may execute the query to determine similarity matches for one or more pairs of search result webpages. System 100 may display similarity matches across pairs of search result webpages (operation 404). System 100 may then receive user input to examine pairs of search result webpages in greater detail and to refine (e.g., editing or selecting) recommended selections (operation 406). Note that FIG. 5 illustrates examples of similarity comparison results for multiple pairs of webpages. FIG. 6 illustrates examples of results of similarity analysis for a pair of webpages to provide initial content recommendation. FIG. 7 illustrates examples of results from comparing the same pair of webpages, which allows the user to view, edit, and select the recommended selections. System 100 may allow the user to post refinements for critique, comment, and further refinement by others (operation 408). System 100 subsequently generates validated content for program delivery system 110, based on the user input (operation 410). System 100 may store the validated content in validated suggestions storage 108.

### Content-Authoring Website

FIG. 5 presents an illustration of a content-authoring website 500 displaying analysis of text chunks across webpage pairs that each match a search query, according to an embodiment. System 100 displays to the user the result of comparing overlap between pairs of webpages that satisfy the search query.

System 100 may analyze the text from two webpages to determine whether the cosine similarity exceeds a predetermined threshold (e.g., cosine similarity of 0.8) when comparing canonically represented sentences. For example, system 100 compares a pair of webpages including a first webpage associated with a first URL 502 against a second webpage associated with a second URL 504. In the depicted example, system 100 determines that there are 12 overlapping sentences, and displays text 506 as "Overlap: 12 sentences." The cosine similarity for the two webpages is 0.8 as indicated in box 508. In some implementations, system 100 may also compare across three or more webpages to determine similarities and extract suggested content.

The user can then choose a pair of webpages for initial content recommendation, as described further with respect to FIGs. 6 and 7.

### Displacing Similarity Analysis for a Pair Of Webpages

FIG. 6 presents an illustration of results of similarity analysis for a pair of webpages to provide initial content recommendation, according to an embodiment. System 100 depicts similarities between two webpages, which is the content overlap between the two webpages. FIG. 6 depicts 12 overlapping sentences as underlined sentences, although different implementations may display overlapping sentences in different ways. For example, some implementations may display a shaded background behind overlapping sentences or use bold text to indicate overlapping sentences. System 100 may determine the initial content recommendation based on the overlapping sentences.

As illustrated in FIG. 6, a user can click on "show/hide report" control 602 or "undo show/hide" control 603 to choose to display or hide a similarity comparison report. The comparison report depicted in FIG. 6 shows that "Doing sit-ups is a quick way to get stronger abdominal muscles" text 604, 606 is a sentence that appears on both webpages. "CLICK PERFORMANCE ADSENSE START" text 608 is only present in one of the websites, and is therefore not depicted (e.g., not underlined) as a sentence that is common to both websites. "Tighten your abdominal muscles gently by drawing in your belly button to your spine" text 610, 612 is also a sentence that is present on both webpages. "Tighten your abdominal muscles gently by drawing in your belly button to your spine" text 614, 616 also is present in both webpages.

### Displayng a Recommended Content Selection

FIG. 7 presents an illustration of displaying a recommended content selection in the context of other contiguous text to receive user selection for content generation, according to an embodiment.

As illustrated in FIG. 7, system 100 display the similarities between two webpages. System 100 also displays, based on determining the initial content recommendation, the recommended content selection 702 so that the user can view and edit the recommended content. For example, a user may edit a recommended content "Place your hand on opposing shoulders, so that your arms are crossed over your chest, or behind your head" text 704. In some implementations, the user can vote for, vote against, or choose the recommended content to be a content suggestion.

### Crowdsourcing and Collaborative Filtering

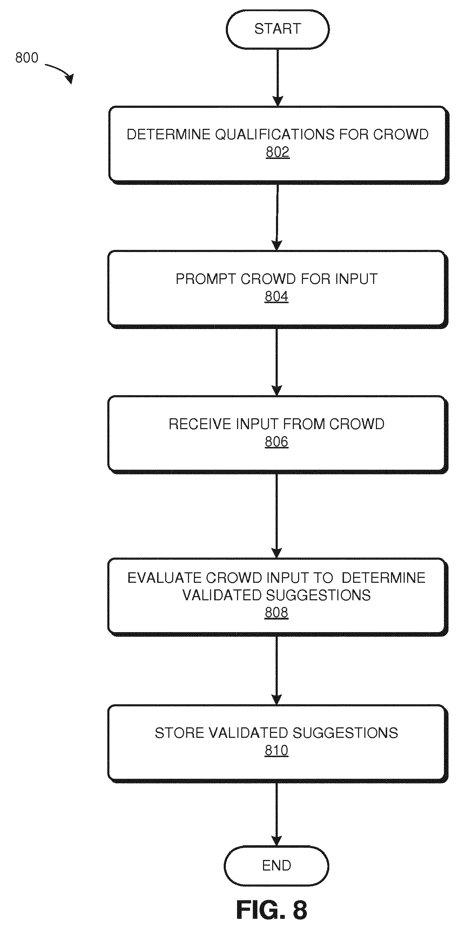

FIG. 8 presents a flowchart 800 illustrating an exemplary process for using crowdsourcing and collaborative filtering to provide validated suggestions, according to an embodiment. FIG. 8 illustrates one possible implementation, and specific details may vary according to implementation.

System 100 may apply crowdsourcing and collaborative filtering methods to identify, assess, and adapt content, including using a crowd to generate, prioritize, critique, and reorder content. System 100 may submit a combination of crowdsourcing tasks to a crowdsourcing system (e.g., Human Intelligence Tasks in Amazon Mechanical Turk). System 100 (e.g., as a content generation system) may assess the results from the crowdsourcing tasks as candidate suggestions or final content suitable for program delivery (e.g., validated suggestions).

In some scenarios, system 100 may extract content from external sources at different levels of granularity, including phrases (e.g., *n*-grams, list elements), sentences, and contiguous sentence sets (e.g., webpages). After extracting content using the methods described herein, system 100 may apply crowdsourcing and collaborative filtering techniques as described below to evaluate and validate content.

During operation, system 100 may initially determine qualifications for the crowd. Note that a crowd is a group of people (e.g., a plurality of users) (operation 802). System 100 may prompt the crowd for input (operation 804). System 100 may solicit feedback from the crowd regarding content or request that the crowd provide input to perform other tasks associated with determining validated suggestions.

System 100 may prompt a crowd (e.g., a set of users) to generate content. For example, given one exemplar of content (e.g., one or more content elements), system 100 may request the crowd to generate another related or unrelated example. Given some attributes needed for content, system 100 may request the crowd to generate another related or unrelated example, including suggestions of a kind such as "what is a good [X]," where X is some item of interest such as snack, exercise, etc., and substitutions for a kind such as "what [exercise] can be substituted for push ups." Also, system 100 may request the crowd to provide a paraphrasing of content, for a given exemplar of content.

System 100 may also prioritize content or content sets by soliciting input from a crowd. Given a set of content examples, system 100 may ask the crowd to select or rank order among the set (e.g., best/worst example, most/least related).

System 100 may also request critique of content from a crowd. Given a set of content, system 100 may solicit input from the crowd on what is missing, wrong, or extra in the set. Given a set of content, system 100 may also request the crowd to reorder or rearrange the content.

System 100 receives input from the crowd (operation 806). In some scenarios, system 100 may generate suggested content for addressing a health-related issue based on the crowd feedback or input. System 100 then evaluates the crowd input to determine validated suggestions (operation 808). System 100 subsequently stores the validated suggestions in validated suggestions storage 108 (operation 810). As part of the evaluating process, system 100 may receive input from one or more users for evaluating the suggested content to validate the suggested content.

### Exemplary Apparatus

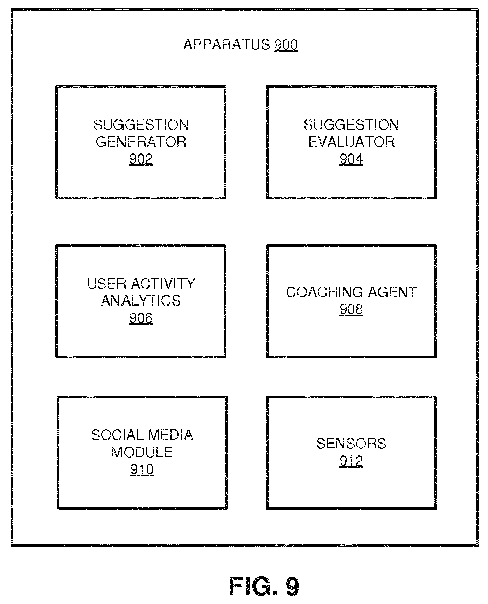

FIG. 9 presents a block diagram illustrating an exemplary apparatus 900 for personalizing healthcare suggestions, in accordance with an embodiment. Apparatus 900 can comprise a plurality of modules which may communicate with one another via a wired or wireless communication channel. Apparatus 900 may be realized using one or more integrated circuits, and may include fewer or more modules than those shown in FIG. 9. Further, apparatus 900 may be integrated in a computer system, or realized as a separate device which is capable of communicating with other computer systems and/or devices.

Apparatus 900 may be implemented as a mobile device, wearable device, a computer with a web-based interface, or a Bluetooth headset with an audio interface. Some implementations may include a server that processes the information and sends the information to the user's device.

Specifically, apparatus 900 can comprise any combination of suggestion generator 902, suggestion evaluator 904, user activity analytics 906, coaching agent 908, social media module 910 and/or sensors 912. Note that apparatus 900 may also include additional modules and data not depicted in FIG. 9, and different implementations may arrange functionality according to a different set of modules. Embodiments of the present invention are not limited to any particular arrangement of modules.

Suggestion generator 902 generates suggested content by accessing external content or any other sources of data. Suggestion evaluator 904 facilitates evaluation of suggested content. User activity analytics 906 analyzes user activity to determine potential health problems and issues, and/or to facilitate activity-based suggestions, and/or to facilitate determining the extent to which the user follows through with suggestions. Coaching agent 908 learns about a team and its members during a challenge, and provides personalized advice, encouragement, and feedback. Social media module 910 allows users to communicate with each other through social media messages and postings. Sensors 912 represents different types of sensors to detect user movement and surrounding environment. For example, sensors 912 may include an accelerometer, a gyroscope, a digital compass, a light sensor, a thermometer, a pedometer, a heart rate monitor, a barometer, GPS sensors, and a microphone.

### Exemplary System

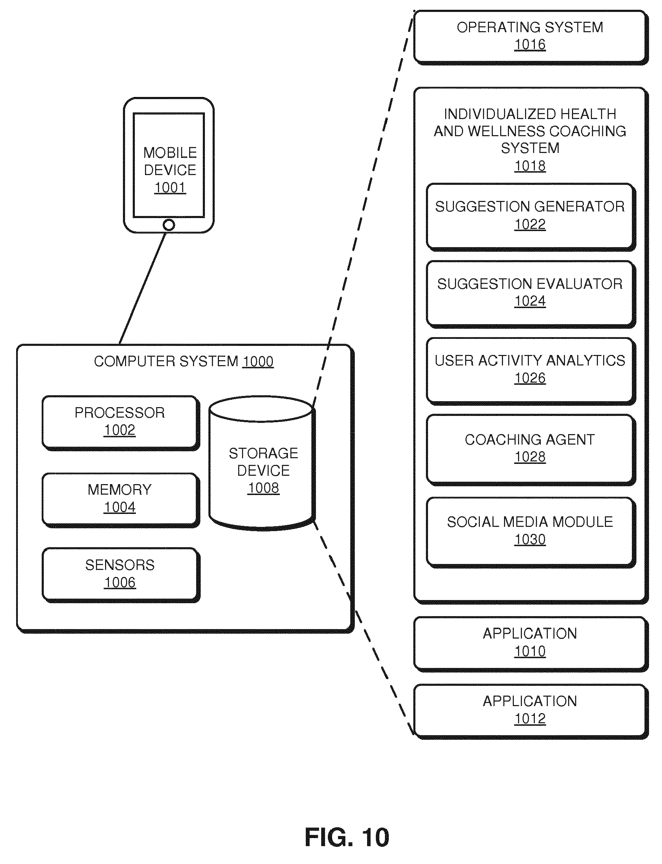

FIG. 10 illustrates an exemplary computer system that may personalize healthcare suggestions, in accordance with an embodiment. A computer and communication system 1000 may be part of a mobile device 1001. In one embodiment, computer system 1000 includes a processor 1002, a memory 1004, sensors 1006, and a storage device 1008.

Sensors 1006 represents different types of sensors to detect user movement and the surrounding environment. For example, sensors 1006 may include an accelerometer, a gyroscope, a digital compass, a light sensor, a thermometer, a pedometer, a heart rate monitor, a barometer, GPS sensors, and a microphone.

Storage device 1008 stores a number of applications, such as applications 1010 and 1012 and operating system 1016. Storage device 1008 also stores code for individualized health and wellness coaching system 1018, which may include components such as suggestion generator 1022, suggestion evaluator 1024, user activity analytics 1026, coaching agent 1028, and social media module 1030.

Suggestion generator 1022 generates suggested content by accessing external content or any other sources of data. Suggestion evaluator 1024 facilitates evaluation of suggested content. User activity analytics 1026 analyzes user activity to determine potential health problems and issues, and/or to facilitate generating and delivering activity-based suggestions, and/or to facilitate determining the extent to which the user follows through with suggestions. Coaching agent 1028 learns about a team and its members during a challenge, and provides personalized advice, encouragement, and feedback. Social media module 1030 allows users to communicate with each other through social media messages and postings.

During operation, one or more applications, such as suggestion generator 1022, are loaded from storage device 1008 into memory 1004 and then executed by processor 1002. While executing the program, processor 1002 performs the aforementioned functions.

The data structures and code described in this detailed description are typically stored on a computer-readable storage medium, which may be any device or medium that can store code and/or data for use by a computer system. The computer-readable storage medium includes, but is not limited to, volatile memory, non-volatile memory, magnetic and optical storage devices such as disk drives, magnetic tape, CDs (compact discs), DVDs (digital versatile discs or digital video discs), or other media capable of storing computer-readable media now known or later developed.

The methods and processes described in the detailed description section can be embodied as code and/or data, which can be stored in a computer-readable storage medium as described above. When a computer system reads and executes the code and/or data stored on the computer-readable storage medium, the computer system performs the methods and processes embodied as data structures and code and stored within the computer-readable storage medium.

Furthermore, methods and processes described herein can be included in hardware modules or apparatus. These modules or apparatus may include, but are not limited to, an application-specific integrated circuit (ASIC) chip, a field-programmable gate array (FPGA), a dedicated or shared processor that executes a particular software module or a piece of code at a particular time, and/or other programmable-logic devices now known or later developed. When the hardware modules or apparatus are activated, they perform the methods and processes included within them.

The foregoing descriptions of various embodiments have been presented only for purposes of illustration and description. They are not intended to be exhaustive or to limit the present invention to the forms disclosed. Accordingly, many modifications and variations will be apparent to practitioners skilled in the art. Additionally, the above disclosure is not intended to limit the present invention.

## Claims

1. A computer-executable method for generating healthcare suggestions, comprising:
extracting, by a computer, data based on a user's communication, which can be between the user and other users or presented online by the user;
identifying a health-related issue from the extracted data;
generating, based on the extracted data, content that indicates a first suggestion corresponding to the health-related issue; and
monitoring the user's communication to generate an additional suggestion that is an improvement over the first suggestion.

2. The method of claim 1, further comprising:
determining qualifications for a plurality of users that can be accessed through a crowdsourcing platform;
soliciting feedback from the plurality of users regarding the health-related issue;
receiving feedback from the plurality of users;
generating, based on the feedback, suggested content for addressing the health-related issue;
receiving input from one or more users for evaluating the suggested content to validate the suggested content; and
storing validated suggestions in a validated suggestion storage.

3. The method of claim 2, further comprising:
presenting a candidate substitution to a user as conversational content; and
receiving the user's vote on whether the user likes the candidate substitution.

4. The method of claim 1, wherein extracting data further comprises:
generating validated suggestions by identifying health problems and extracting solutions and health advice with ongoing monitoring of one of sensor data, website forums, instant messages, websites, and social media communication.

5. The method of claim 1, further comprising:
generating a healthcare question and posting the healthcare question on a website forum to solicit responses from forum users;
monitoring responses to the healthcare question on the website forum; and
generating a suggested solution addressing the healthcare question based on the responses to the posted healthcare question.

6. A computer-readable storage medium storing instructions that when executed by a computer cause the computer to perform a method for generating healthcare suggestions, the method comprising:
extracting, by a computer, data based on a user's communication, which can be between the user and other users or presented online by the user;
identifying a health-related issue from the extracted data;
generating, based on the extracted data, content that indicates a first suggestion corresponding to the health-related issue; and
monitoring the user's communication to generate an additional suggestion that is an improvement over the first suggestion.

7. The computer-readable storage medium of claim 6, wherein the method further comprises:
determining qualifications for a plurality of users that can be accessed through a crowdsourcing platform;
soliciting feedback from the plurality of users regarding the health-related issue;
receiving feedback from the plurality of users;
generating, based on the feedback, suggested content for addressing the health-related issue;
receiving input from one or more users for evaluating the suggested content to validate the suggested content; and
storing validated suggestions in a validated suggestion storage.

8. The computer-readable storage medium of claim 6, wherein the method further comprises:
presenting a candidate substitution to a user as conversational content; and
receiving the user's vote on whether the user likes the candidate substitution.

9. A computing system for generating healthcare suggestions, the
system comprising:
one or more processors,
a computer-readable medium coupled to the one or more processors having instructions stored thereon that, when executed by the one or more processors, cause the one or more processors to perform operations comprising:
extracting, by a computer, data based on a user's communication, which can be between the user and other users or presented online by the user;
identify a health-related issue from the extracted data;
generating, based on the extracted data, content that indicates a first suggestion corresponding to the health-related issue; and
monitoring the user's communication to generate an additional suggestion that is an improvement over the first suggestion.

10. The computing system of claim 9, wherein the operations further comprise:
determining qualifications for a plurality of users that can be accessed through a crowdsourcing platform;
soliciting feedback from the plurality of users regarding the health-related issue;
receiving feedback from the plurality of users;
generating, based on the feedback, suggested content for addressing the health-related issue;
receiving input from one or more users for evaluating the suggested content to validate the suggested content; and
storing validated suggestions in a validated suggestion storage.
